# EUROPÄISCHE PATENTANMELDUNG

(11) **EP 0 832 846 A2**
(43) Veröffentlichungstag der Anmeldung: **01.04.1998**
(21) Anmeldenummer: 97116898.4
(22) Anmeldetag: 29.09.1997
(51) Int. Cl.: C01B 15/037, C01B 15/01, C08K 3/00, C02F 1/72, A01N 59/00

(54) **Polymer-Wasserstoffperoxid-komplexe**

(30) Priorität: 30.09.1996 DE 19640365
(71) Anmelder: BASF AKTIENGESELLSCHAFT, 67056 Ludwigshafen (DE)
(72) Erfinder: Breitenbach, Jörg, Dr., 68199 Mannheim (DE); Fussnegger, Bernhard, Dr., 67489 Kirrweiler (DE); Lang, Siegfried, Dr., 67071 Ludwigshafen (DE); Reich, Hans-Bernd, 67141 Neuhofen (DE)
(74) Vertreter: Kinzebach, Werner, Dr.

(57) **Zusammenfassung**

Die vorliegende Erfindung betrifft Polymerkomplexe, die Wasserstoffperoxid, ein zur Komplexbildung mit Wasserstoffperoxid geeignetes Polymer und wenigstens ein Metallkolloid und/oder Metallsalz enthalten sowie ein Verfahren zu ihrer Herstellung.

Die vorliegende Erfindung betrifft weiterhin die Verwendung der Polymerkomplexe in bakteriziden Mitteln, Desinfektionssystemen, haarkosmetischen Mitteln, als radikalische Initiatoren chemischer Reaktionen sowie Mittel, die diese Komplexe enthalten.

## Beschreibung

Die vorliegende Erfindung betrifft Polymerkomplexe, die Wasserstoffperoxid, ein zur Komplexbildung mit Wasserstoffperoxid geeignetes Polymer und wenigstens ein Metallkolloid und/oder Metallsalz enthalten.

Wasserstoffperoxid findet als Oxidations- und Bleichmittel, Desinfektionsmittel, Mittel zur Desodorierung, aber auch als Radikalstarter für chemische Prozesse, beispielsweise für Polymerisationsreaktionen zahlreiche kommerzielle und industrielle Anwendungen (siehe Römpp Chemie-Lexikon, 9. Aufl. "Wasserstoffperoxid" und dort zitierte Literatur). Insbesondere im Desinfektionsbereich gewinnt es zunehmend an Bedeutung, da es umweltverträglicher ist als halogenhaltige Desinfektionsmittel.

Wasserstoffperoxid zersetzt sich jedoch in merklichem Maße durch Einwirkung von Wärme oder Licht oder in Gegenwart von Verunreinigungen wie Staub, verschiedenen Metallsalzen sowie alkalisch reagierenden Substanzen. Zwar ist die Zersetzungsreaktion bei den meisten Anwendungen erwünscht, die Lager- und Gebrauchsfähigkeit wird jedoch eingeschränkt, da der Gehalt an Wasserstoffperoxid abnimmt.

Neben der Stabilisierung durch klassische Stabilisatoren wie Silikate, Phosphate, Gelatine, Dextrine und Komplexbildner wurde auch die Stabilisierung von Wasserstoffperoxid durch kolloidale Lösungen von Metallen, insbesondere durch Silberkolloide (DE-A 3 620 609, EP-B 596 908) beschrieben.

Wasserstoffperoxid kann auch in Form von Komplexen mit Polymeren, vorzugsweise Polyvinylpyrrolidonen, stabilisiert werden. Solche Komplexe sind bekannt und beispielsweise in US-A 3,376,110, US-A 3,480,557, US-A 5,077,047, US-A 5,108,742, WO-A 91/07184 und WO-A 92/17158 beschrieben. Diese Komplexe sind in der Regel feste, stabile Pulver, die sich leicht handhaben lassen und in eine Vielzahl von Zubereitungen eingearbeitet werden können. Die Geschwindigkeit, mit der Wasserstoffperoxid an das umgebende Medium abgegeben wird, hängt von der Bindungsstärke des Wasserstoffperoxids an den polymeren Träger ab. Die Geschwindigkeit, mit der das Wasserstoffperoxid seine chemische Wirkung entfaltet, hängt hingegen von den im Medium vorhandenen Zersetzungskatalysatoren (Staubpartikel, basische Verunreinigungen, Metallspuren) ab.

Es ist weiterhin bekannt, daß Polyvinylpyrrolidon als Schutzpolymer für metallische Kolloid-Lösungen, beispielweise von Kupfer, Silber (Hirai et al., Makromol. Chem. Rapid Commun. 5 (1984) 381), Palladium, Gold, Rhodium oder Platin, eingesetzt werden kann. Esumi et al. beschreiben die Herstellung kolloidaler Silberlösungen in Anwesenheit von Vinylalkohol und N-Vinylpyrrolidon (J. Appl. Polym. Sci. 44 (1992) 1003) oder Polyvinylpyrrolidon-Homopolymeren (Hirai et al. J. Macromol. Sci. Chem. A13 (1979) 633). Auch bimetallische Kolloide, insbesondere zum Einsatz als Katalysatoren, wurden von Wang et al. (Polymer Bulletin 25 (1991) 139) beschrieben.

Es ist ferner bekannt, daß Silberionen in Form von Silbersalzen toxikologisch unbedenkliche Antiseptika mit breitem Wirkspektrum darstellen. So wird 1 %-ige Silbernitratlösung Neugeborenen zur Credé-Prophylaxe (Vermeidung von Gonoblennorrhoe) direkt nach der Geburt in den Bindehautsack appliziert.

Der vorliegenden Erfindung liegt die Aufgabe zugrunde, neben der Freisetzung des Wasserstoffperoxids aus solchen Komplexen auch seine Zersetzung zu kontrollieren. Außerdem sollen die Komplexe ein breites Wirkungsspektrum besitzen.

Diese Aufgabe konnte überraschenderweise gelöst werden durch polymergebundenes Wasserstoffperoxid, wobei im Polymer zusätzlich ein Metallkolloid oder Metallsalz gebunden ist.

Die vorliegende Erfindung betrifft daher Polymerkomplexe, die
a) Wasserstoffperoxid,
b) ein zur Komplexbildung mit Wasserstoffperoxid geeignetes Polymer und
c) wenigstens ein Metallkolloid und/oder Metallsalz umfassen,
wobei die Bestandteile a) und b) einen Komplex bilden.

Die Erfindung betrifft weiterhin Verfahren zur Herstellung dieser Polymerkomplexe.

Unter C₁-Cₙ-Alkyl versteht im folgenden man lineare, verzweigte oder cyclische Alkylgruppen mit 1 bis n Kohlenstoffatomen. Beispiele sind Methyl, Ethyl, n-Propyl, i-Propyl, n-Butyl, 2-Butyl, i-Butyl, t-Butyl, n-Hexyl, 2-Hexyl, 2-Ethylhexyl oder n-Decyl, Cyclopentyl oder Cyclohexyl. Unter C₁-Cₙ-Alkylen versteht man lineare oder verzweigte Alkyleneinheiten, beispielsweise Methylen, Ethylen, Ethyliden, 1,1-, 1,2-, 1,3-, 2,2-Propylen, 1,1-, 1,2-, 1,3-, 1,4-, 2,2-, 2,3-Butylen. Arylgruppen sind Phenyl oder Naphthyl, die gegebenenfalls 1 bis 3 C₁-C₄-Alkylgruppen oder Halogenatome als Substituenten tragen.

Bei dem Bestandteil b) handelt es sich vorzugsweise um ein Homo- oder Copolymer eines oder mehrerer N-Vinyllactame. Bevorzugte N-Vinyllactame sind N-Vinylpyrrolidon, N-Vinylpiperidon, N-Vinylcaprolactam, N-Vinylmorpholin-3-on, N-Vinyloxazolidin-4-on und deren Mischungen. Als Comonomere kommen insbesondere N-Vinylheterocyclen in Frage, z.B. Vinylpyridine oder Vinylimidazole, die gegebenenfalls einen oder mehrere C₁-C₄-Alkylreste oder Phenylreste tragen. Als Beispiele seien genannt: N-Vinylimidazol sowie 2-Methyl-1-vinylimidazol, 4-Methyl-1-vinylimidazol, 5-Methyl-1-vinylimidazol, 2-Ethyl-1-vinylimidazol, 2-Propyl-1-vinylimidazol, 2-Isopropyl-1-vinylimidazol, 2-Phenyl-1-vinylimidazol, 2-Vinylpyridin, 4-Vinylpyridin sowie 2-Methyl-5-vinylpyridin. Weiterhin können eingesetzt werden C₁-C₈-Alkylvinylether, z.B. Methylvinylether, Ethylvinylether, n-Propylvinylether, i-Propylvinylether, n-Butylvinylether, i-Butylvinylether, t-Butylvinylether, 2-Ethylhexylvinylether, Vinylester von C₁-C₁₀-Alkyl- oder C₆-C₁₀-Arylcarbonsäuren, z.B. Vinylacetat, Vinylpropionat, Vinylbutyrat, Vinylhexanoat, Vinyl-2-ethylhexanoat, Vinyldecanoat, Vinyllaurat, Vinylstearat oder Vinylbenzoat. Weiterhin kommen Ester der Acrylsäure oder der Methacrylsäure mit C₁-C₁₂-Alkanolen, vorzugsweise C₁-C₄-Alkanolen in Frage. Beispiele hierfür sind Methyl(meth)acrylat, Ethyl(meth)acrylat, n-Butyl(meth)acrylat. Weitere mögliche Comonomere sind konjugierte C₄-C₈-Diene wie Butadien oder Isopren, Vinylaromaten wie Styrol, α-Methylstyrol oder Vinyltoluole und kationisch modifizierte Vinylmonomere. Bei letzteren handelt es sich beispielsweise um monoethylenisch ungesättigte C₃-C₅-Carbonsäureester mit Aminoalkoholen der Formel 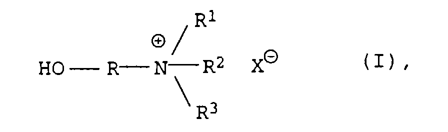 in der R für C₂-C₅-Alkylen steht, R¹, R², R³ unabhängig voneinander für H, CH₃, C₂H₅, C₃H₇ stehen und X^{⊖} ein Anion bedeutet. Geeignet sind außerdem Amide dieser Carbonsäuren, die sich von Aminen der Formel 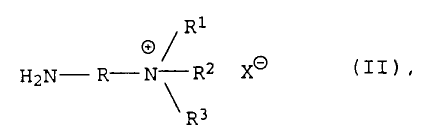 ableiten. Die Substituenten in Formel II und X^{⊖} haben die gleiche Bedeutung wie in Formel I. Geeignete Carbonsäuren sind beispielsweise Acrylsäure, Methacrylsäure, Crotonsäure, Maleinsäure(anhydrid), Fumarsäure und Itaconsäure. Als kationisch modifizierte Vinylmonomere eignen sich auch Salze oder Quaternisierungsprodukte von N-Vinylimidazol und 1-Vinyl-2-methylimidazol.

Die für die erfindungsgemäßen Polymerkomplexe verwendeten Polymerisate enthalten die genannten N-Vinyllactame in Mengen oberhalb 20 Gew.-%, vorzugsweise 30 bis 99 Gew.-% und insbesondere 35 bis 80 Gew.-% und die Comonomere in Mengen von bis zu 80 Gew.-%, vorzugsweise 1 bis 70 Gew.-% und insbesondere 20 bis 65 Gew.-% einpolymerisiert, wobei die Gewichtsteile jeweils auf das Polymerisat bezogen sind.

Weiterhin können diese Polymerisate in Mengen von bis zu 20 Gew.-%, vorzugsweise bis zu 5 Gew.-% und insbesondere 0,1 bis 3 Gew.-%, jeweils bezogen auf das Polymerisat, vernetzend wirkende Monomere mit einpolymerisiert enthalten. Geeignete Vernetzer sind in der Regel Verbindungen, die wenigstens 2, nicht konjugierte, ethylenisch ungesättigte Doppelbindungen im Molekül enthalten. Beispiele hierfür sind N,N'-Methylenbisacrylamid, Polyethylenglykoldiacrylate und Polyethylenglykoldimethacrylate, Trimethylolpropantriacrylat, Trimethylolpropantrimethacrylat, Ethylenglykoldiacrylat, Propylenglykoldiacrylat, Butandioldiacrylat, Hexandioldiacrylat, Hexandioldimethacrylat, Diacrylate und Dimethacrylate von Blockcopolymerisaten aus Ethylenoxid und Propylenoxid, zweifach bzw. dreifach mit Acrylsäure oder Methacrylsäure veresterte mehrwertige Alkohole wie Glycerin oder Pentaerythrit, Triallylamin, Tetraallylethylendiamin, Trimethylolpropandiallylether, Pentaerythrittriallylether, N,N'-Divinylethylenharnstoff und/oder N,N'-Divinylpropylenharnstoff. Weiterhin kommen in Frage Divinylaromaten wie beispielsweise Divinylbenzol, aber auch Dicyclopentadien, Vinyl(meth)acrylat, Vinylnorbornen, Tricyclodecenyl(meth)acrylat.

In einer bevorzugten Ausführungsform werden wasserlösliche Polymerisate eingesetzt. Hierbei bedeutet "wasserlöslich", daß die erfindungsgemäß verwendeten Polymerisate bei 20°C eine Löslichkeit von wenigstens 0,5 g, vorzugsweise wenigstens 2 g und insbesondere wenigstens 5 g in 100 g Wasser aufweisen. Bevorzugte Comonomere sind in diesem Fall Vinylacetat, Vinylpropionat, Methylacrylat, Ethylacrylat, n-Propylacrylat, n-Butylacrylat, Methylmethacrylat, Ethylmethacrylat, Acrylnitril, Methacrylnitril und Vinylimidazol. Ebenfalls geeignet sind Copolymerisate der N-Vinyllactame untereinander. Besonders bevorzugt werden Copolymere aus N-Vinylpyrrolidon und N-Vinylcaprolactam, N-Vinylpyrrolidon und Vinylacetat sowie Homopolymere des N-Vinylpyrrolidons. Diese wasserlöslichen Homo- und Copolymerisate weisen K-Werte nach Fikentscher (siehe Cellulose-Chemie 13, S. 48-64 und S. 71-94 (1932)) im Bereich von 10 bis 110, vorzugsweise 20 bis 100 auf.

Die Herstellung der wasserlöslichen Polymerisate auf N-Vinyllactam-Basis ist an sich bekannt und beispielsweise in DE-A 22 18 935 oder der älteren Anmeldung P 196 09 864.5 beschrieben. Ihre Herstellung erfolgt vorzugsweise durch radikalische Lösungspolymerisation in einem wäßrigen oder alkoholischen Lösungsmittel, beispielsweise in Wasser, Methanol, Ethanol, i-Propanol oder deren Mischungen.

Als Initiatoren für die radikalische Polymerisation kommen insbesondere jene in Frage, die für die radikalische Polymerisation in wäßriger Lösung geeignet sind. Besonders geeignet sind aliphatische oder cycloaliphatische Azoverbindungen, z.B. 2,2'-Azobisisobutyronitril), 2,2'-Azobis(2-methylbutyronitril), 2,2'-Azobis(2,4-dimethylvaleronitril), 1,1'-Azobis(1-cyclohexancarbonitril), (2-Carbamoylazo)isobutyronitril, 4,4'-Azobis(4-cyanovaleriansäure) und deren Alkalimetall- und Ammoniumsalze, Dimethyl-2,2'-azobisisobutyrat, 2,2'-Azobis[2-(2-imidazolin-2-yl)propan], 2,2'-Azobis(2-amidinopropan) und die Säureadditionssalze der beiden letztgenannten Verbindungen. Weiterhin kommen als Initiatoren Wasserstoffperoxid, Hydroperoxide in Kombination mit geeigneten Reduktionsmitteln und Persalze in Frage. Geeignete Hydroperoxide sind beispielsweise t-Butylhydroperoxid, t-Amylhydroperoxid, Cumolhydroperoxid und Pinanhydroperoxid, jeweils in Kombination mit beispielsweise einem Salz der Hydroxymethansulfinsäure, einem Eisen(II)-Salz oder Ascorbinsäure. Geeignete Persalze sind insbesondere Alkalimetallperoxodisulfate. Die verwendete Initiatormenge, bezogen auf die Monomere, liegt im Bereich von 0,02 bis 15 mol-%, vorzugsweise 0,05 bis 3 mol-%.

Die Polymerisation wird üblicherweise bei einem neutralen pH-Wert im Bereich von 5 bis 9 durchgeführt. Sofern notwendig, wird der pH-Wert durch Zugabe einer Base wie Ammoniak oder einer Säure wie HCl oder eines Puffersystems eingestellt bzw. aufrechterhalten. Sind niedrige Molekulargewichte erwünscht, kann die Reaktion auch in Gegenwart einer das Molekulargewicht der Polymerisate regelnden Verbindung durchgeführt werden. Hierbei handelt es sich beispielsweise um Aldehyde wie Formaldehyd, Acetaldehyd, Propionaldehyd oder Allylverbindungen wie Allylalkohol. Weiterhin können Regler eingesetzt werden, die Schwefel in organisch gebundener Form enthalten. Hierbei handelt es sich beispielsweise um Butylmercaptan, n-Hexylmercaptan, n-Dodecylmercaptan, wasserlösliche Verbindungen wie beispielsweise Hydrogensulfite, Disulfite, Ethylthioglykolat, Cystein, 2-Mercaptoethanol, Mercaptoessigsäure, 3-Mercaptopropionsäure, Thioglycerin, Diethanolsulfid, Thiodiglykol, Thioharnstoff oder Dimethylsulfoxid.

Die entstandenen Polymerisat-Lösungen weisen im allgemeinen Feststoffgehalte im Bereich von 3 bis 70 Gew.-%, bevorzugt 30 bis 60 Gew.-% auf. Sie können, so wie sie bei der Polymerisation anfallen, ohne weitere Isolierung oder Behandlung für die erfindungsgemäße Verfahren eingesetzt oder aber durch Fällung oder Entfernung des Lösungsmittels als Trockensubstanz isoliert werden.

In einer anderen bevorzugten Ausführungsform werden unlösliche Polymerisate eingesetzt. Solche Polymerisate werden erhalten, wenn man die Monomere in Gegenwart eines der obengenannten Vernetzer polymerisiert. Die Polymerisate können jedoch auch nachträglich durch physikalische Einflüsse, wie z.B. Strahlung oder durch chemische Reaktion mit einer bi- oder polyfunktionellen Verbindung, die mit den in den Polymerisaten enthaltenen funktionellen Gruppen reagieren kann, vernetzt und damit unlöslich gemacht werden. Solche Verfahren sind dem Fachmann bekannt und in der Literatur beschrieben. Eine besonders bevorzugte Ausführungsform der unlöslichen Polymerisate sind die sogenannten Popcorn-Polymerisate (Römpp, Chemie-Lexikon, 9. Aufl. "Popcorn Polymerisate" und dort zitierte Literatur). Die Herstellung solcher Popcorn-Polymerisate ist beispielsweise in EP-A 88 964 und EP-A 438 713 beschrieben. Sie werden in der Regel durch Substanz-, Lösungs- oder Fällungspolymerisation der Monomere, vorzugsweise in Gegenwart geringer Mengen eines Vernetzers (0,1-4 Gew.-%, bezogen auf Monomere) hergestellt.

Die erfindungsgemäßen Polymerkomplexe enthalten als Metallkomponente c) vorzugsweise ein Metallsalz oder Metallkolloid des Kupfers, Silbers, Golds, Rhodiums, Iridiums, Palladiums oder Platins. Besonders bevorzugt sind Kolloide des Kupfers oder des Silbers, insbesondere des Silbers. Geeignete Silbersalze sind beispielsweise Silbernitrat, Silberacetat, Silberlactat, Silberphosphat, Silberchlorid, Silberbromid, Silberhydroxid, Silbercarbonat, Silberoxid, Silberperiodat oder der Natriumchlorid-Silberchloridkomplex (Na [AgCl₂]). Silberkolloide sind beispielsweise dadurch erhältlich, daß man wäßrige Lösungen eines geeigneten Silbersalzes mit einem Reduktionsmittel wie Wasserstoff, Ascorbinsäure, Ribose, Glucose, Hydrazin, einem Aldehyd oder einem Alkohol behandelt (siehe Römpp, Chemie-Lexikon, 9. Aufl. "Kolloide"). Die erfindungsgemäßen Komplexe enthalten Wasserstoffperoxid in Mengen von vorzugsweise 0,5 bis 40 Gew.-%, besonders bevorzugt 5 bis 23 Gew.-% und insbesondere 6 bis 15 Gew.-%, jeweils bezogen auf den fertigen Polymerkomplex. Der Polymerisatgehalt beträgt in der Regel 55 bis 99,5 Gew.-%, vorzugsweise 74 bis 95 Gew.-% und insbesondere 83 bis 94 Gew.-%. Der Metallgehalt in den Polymerkomplexen beträgt in der Regel 0,001 bis 5 Gew.-%, vorzugsweise 0,005 bis 3 Gew.-% und insbesondere 0,01 bis 2 Gew.-%.

Die Herstellung der erfindungsgemäßen Polymerkomplexe kann auf verschiedene Arten und Weisen erfolgen und hängt von der Art der verwendeten Polymere und der Metallkomponente ab. Bei Verwendung löslicher Polymere hat sich die Sprühtrocknung oder Sprühgranulation bewährt. Hierbei werden vorzugsweise wäßrige Lösungen der Polymere mit Lösungen von Wasserstoffperoxid mit Lösungen der Metallsalze bzw. Dispersionen der Metallkolloide unter Einsatz von Mehrstoffdüsen gemeinsam sprühgetrocknet oder granuliert. Lösungen können auch zuvor vermischt worden sein. Auch ist es möglich, durch Metallsalze oder Kolloide stabilisierte Wasserstoffperoxid-Lösungen einzusetzen. In einer anderen Variante wird ein Metallkolloid in Gegenwart eines Polymers aus einer wäßrigen Lösung durch eines der genannten Reduktionsmittel abgeschieden. Über die Abscheidung von Metallkolloiden in Gegenwart von Vinylpyrrolidon-Polymerisaten wurde in der Literatur berichtet, siehe oben. Die erhaltene Dispersion wird dann zusammen mit einer Wasserstoffperoxid-Lösung sprühgetrocknet. Auch hier ist es möglich, die Lösungen zuvor zu vermischen. Die verwendeten Polymerlösungen können durch Auflösen des Polymers in einem geeigneten Lösungsmittel, vorzugsweise einem wäßrig alkoholischen oder wäßrigen Lösungsmittel hergestellt werden. Auch ist es möglich, die bei der Polymerisation anfallenden Lösungen direkt zu verwenden. Im erfindungsgemäßen Verfahren wird Wasserstoffperoxid in Form 20 bis 70 gew.%-iger, vorzugsweise 30 bis 60 gew.%-iger wäßriger Lösungen eingesetzt.

Die Verfahren zur Sprühtrocknung oder Sprühgranulierung sind dem Fachmann bekannt. Auch im vorliegenden Fall kann die Gewinnung der festen Polymerkomplexe in Sprühtürmen herkömmlicher Bauart erfolgen. Als Trocknungsgase werden inerte Gase wie beispielsweise Stickstoff verwendet, die im Gegenstrom oder vorzugsweise im Gleichstrom mit den Flüssigkeitstropfen durch den Trocknungsturm geleitet werden. Die Turmeingangstemperatur des Gases liegt in der Regel bei 60 bis 180°C, vorzugsweise bei 100 bis 160°C, und die Turmausgangstemperatur bei 40 bis 100°C, vorzugsweise bei 60 bis 90°C. Der Druck liegt in der Regel im Bereich von 0,6 bis 1,5 bar, vorzugsweise erfolgt die Trocknung bei Normaldruck. Der entstandene Feststoff kann in üblicher Weise von dem Gasstrom, beispielsweise durch ein Zyklon, abgetrennt werden. Man erhält auf diese Weise ein frei fließendes Pulver mit einem Restlösemittelgehalt < 7,5 Gew.-%, bezogen auf den fertigen Polymerkomplex. Die Teilchengröße im entstandenen Pulver liegt im allgemeinen bei 10 bis 150 µm, bei Sprühgranulation können Teilchengrößen von bis zu 450 µm erreicht werden.

Eine andere Ausführungsform der erfindungsgemäßen Komplexe besteht darin, daß man die unlöslichen Formen der N-Vinyllactam-Polymerisate im Wirbelbett mit Metallsalz-Lösung bzw. Metallkolloid und Wasserstoffperoxid umgesetzt wird. Auch hier kann die Metallkomponente zuvor mit der Wasserstoffperoxid-Lösung vermischt werden.

In einer weiteren Ausführungsform weisen die erfindungsgemäßen Komplexe einen schalenförmigen Aufbau auf. Dieser schalenförmige Aufbau wird dadurch erreicht, daß man die obengenannten Bestandteile oder Kombinationen dieser Bestandteile in einer geeigneten Apparatur, beispielsweise einem Dragierkessel oder einem Wirbelschichtgranulator, sukzessive auf einen festen Träger aufträgt. Auch diese Verfahren sind dem Fachmann im Prinzip bekannt. Bei diesem festen Träger handelt es sich um anorganische Oxide wie Titandioxid, Aluminiumoxid, Siliciumdioxid, Silikate, Alumosilikate, organische Trägermaterialien wie Zellulose, Stärke, unlösliche Polymerisate, vorzugsweise solche, die zur Komplexbildung mit Wasserstoffperoxid geeignet sind, insbesondere solche auf Polyvinyllactambasis. Der letztgenannte Träger kann bei der Herstellung der erfindungsgemäßen Komplexe auch als Komponente b) dienen.

Die Herstellung der erfindungsgemäßen, schichtförmig aufgebauten Polymerkomplexe geschieht dadurch, daß man auf den Träger in einer der obengenannten Apparaturen die Lösungen oder Suspensionen der Komponenten a) und c) und gegebenenfalls b) in der für den jeweils gewünschten Aufbau notwendigen Reihenfolge unter den Bedingungen, wie sie für die Polymerkomplexpulver beschrieben worden sind, aufsprüht. Dieser Vorgang kann gegebenenfalls wiederholt werden, bis die gewünschten Konzentrationsverhältnisse der Komponenten eingestellt sind. Die Komponenten können über Mehrstoffdüsen gleichzeitig oder aber sukzessive in beliebiger Reihenfolge aufgesprüht werden.

Soll eine pH-Wert abhängige Freisetzung des Wasserstoffperoxids und des Metalls erfolgen, können die Komplexe mit einem polymeren Filmbildner, der bei einem bestimmten pH-Wert in Lösung geht oder quillt, überzogen werden. Durch geeignete Wahl dieser Filmbildner kann eine Freisetzung sowohl im sauren wie im alkalischen Milieu initiiert werden. Solche Filmbildner können bei schalenförmig aufgebauten Komplexen auch die einzelnen Schichten gegeneinander abgrenzen, so daß z.B. eine erste Schicht bei einem neutralen pH den Wirkstoff freisetzt und eine innere Schicht erst oberhalb oder unterhalb eines bestimmten pH-Wertes. Geeignete polymere Filmbildner sind aus der Galenik bekannt. Dabei handelt es sich beispielsweise um Hydroxypropylcellulose, Hydroxypropylmethylcellulose, Vinylpyrrolidon-Vinylacetatcopolymere, Celluloseacetatphthalat, Hydroxypropylmethylcellulosephthalat, Schellack, Copolymerisate von Acrylsäure oder Methacrylsäure mit (Meth)acrylsäureestern, z.B. Copolymere aus Ethylacrylat und Methacrylsäure (z.B. Kollicoat® MAE 30D oder Endragile®) oder Copolymerisate von Dimethylaminomethacrylsäure mit neutralen Methacrylsäureestern.

Bevorzugte Ausführungsformen der erfindungsgemäßen schalenförmigen Komplexe sind wie folgt aufgebaut:
- Kern: Polymer b) mit Komponente a); Schale: Polymer b) mit Komponente c);
- Kern: Polymer b) mit Komponente a); Schale 1: polymerer Filmbildner (s.o.), Schale 2: Polymer b) mit Komponente c);
- Kern: Polymer b) mit Komponente c); Schale 1: polymerer Filmbildner (s.o.), Schale 2: Polymer b) mit Komponente a).
Ebenfalls denkbar sind schalenförmige Aufbauten, die als Kern Komponente b) mit Komponente a) und/oder Komponente c) enthalten und als Schale einen polymeren Filmbildner aufweisen.

Bei der Herstellung der erfindungsgemäßen Polymerkomplexe, sei es als Polymerpulver oder als schalenförmiges Granulat, können weitere Bestandteile zugegen sein, die die Verarbeitung der erfindungsgemäßen Polymerkomplexe vereinfachen oder deren Wirkungsspektrum erweitern. Beispielsweise kann man bei der Herstellung der erfindungsgemäßen Komplexe Tenside zusetzen, die anschließend im Komplex verbleiben. Diese können in Kontakt mit Keimen die Wirkung des eigentlichen Desinfektions-Systems Wasserstoffperoxid/Metall/Polymer erhöhen und als Lösungsvermittler bzw. Netzmittel dienen. Geeignete Tenside können sowohl kationischer, anionischer als auch nichtionischer Natur sein. Beispiele hierfür sind Natriumdodecylsulfat, Dodecyltrimethylammoniumbromid, Dimethylalklybenzylammoniumchlorid, Polysorbatfettsäureester sowie ethoxylierte Mono-, Di- und Trialkylphenole (EO-Grad: 3 bis 50, Alkylrest:C₄-C₉), ethoxylierte Fettalkohole (EO-Grad: 3 bis 50, Alkylrest C₈-C₃₆), sowie Alkali- und Ammoniumsalze von Alkylsulfaten (Alkylrest: C₈-C₁₂), von Schwefelsäurehalbestern ethoxylierter Alkanole (EO-Grad: 4 bis 30, Alkylrest: C₁₂-C₁₈) und ethoxylierter Alkylphenole (EO-Grad: 3 bis 50, Alkylrest: C₄-C₉), von Alkylsulfonsäuren (Alkylrest: C₁₂-C₁₈) und von Alkylarylsulfonsäuren (Alkylrest: C₉-C₁₈). Bevorzugte Emulgatoren sind Natriumdodecylsulfat und Polysorbatfettsäureester.

Geeignete Substanzen, die das Wirkungsspektrum der erfindungsgemäßen Polymerkomplexe erhöhen, sind beispielsweise Aldehyde und α-Hydroxycarbonsäuren. Beispiele für Aldehyde sind Glutaraldehyd und Glyoxal. Unter den α-Hydroxycarbonsäuren werden vorzugsweise solche ausgewählt, die bei topischer Anwendung pharmazeutisch verträglich sind. Beispiele hierfür sind Glykolsäure, Milchsäure, Hydroxyoctansäure, Äpfelsäure, Brenztraubensäure und Zitronensäure. Ebenfalls geeignet sind Hydroxyarylcarbonsäuren, beispielsweise Salicylsäure, 3-Hydroxybenzoesäure, 4-Hydroxybenzoesäure, 2,4-Dihydroxybenzoesäure, 3,4-Dihydroxybenzoesäure und 3,5-Dihydroxybenzoesäure. Die genannten Verbindungen werden vermutlich ebenfalls vom Polymerisat komplex gebunden (siehe D. Horn et al., J.Pharm.Sci., 71, 1982, S. 1021-1026).

Die erfindungsgemäßen Komplexe sind als Feststoffe bei Raumtemperatur über einen langen Zeitraum hinweg ohne Verlust an Wasserstoffperoxid haltbar. Die Kombination von Wasserstoffperoxid, polymerem Träger und stabilisierendem Metallsalz oder -kolloid führt in den erfindungsgemäßen Verwendungsformen zu einer langsamen Freisetzung des Wasserstoffperoxids und zu seinem kontrollierten Zerfall. Hinsichtlich der desinfizierenden Wirkung der erfindungsgemäßen Komplexe bieten die erfindungsgemäßen Komplexe gegenüber den halogenhaltigen Desinfektionsmitteln, z.B. den bekannten Iod-Polyvinylpyrrolidion-Komplexen, den Vorteil der Farblosigkeit und Geschmacklosigkeit. Die Halogenkomplexe können zudem Hautreizungen hervorrufen. Gegenüber den reinen Polymer-Wasserstoffperoxid-Komplexen ist ihr Wirkungsspektrum vergrößert, da auch Metallkolloide und ihre Ionen breitgefächerte bakterizide Eigenschaften besitzen.

Insbesondere die bakterizide Wirkung der erfindungsgemäßen Komplexe ermöglicht eine Vielzahl von Anwendungen. Da die erfindungsgemäßen Komplexe in der Regel Einsatzstoffe beinhalten, die pharmazeutisch anerkannt sind, und da sie sich den meisten pharmazeutisch verwendeten Formulierungsbestandteilen gegenüber inert zeigen, bestehen hinsichtlich ihrer Formulierung in pharmazeutischen Mitteln kaum Beschränkungen. Als Formulierungsbestandteile werden entsprechend der gewünschten Zubereitungsform die üblichen pharmazeutischen oder kosmetischen Träger und Hilfsmittel verwendet. Als Formulierungsbestandteile können beispielsweise Alkohole wie Ethanol, Propanol, Isopropanol, Phenoxyethanol, Phenoxy-1- und Phenoxy-2-propanol, Polyole wie Propylenglykol, Glycerin oder Polyethylenglykole, Silicone, Ester oder Glyceride von Fettsäuren, beispielsweise Isopropylmyristat, Myricylcerotinoat, Cetylpalmitat, Glyceride der Palmitinsäure, Stearinsäure, Linolsäure, Linolensäure oder Ölsäure, Phospholipide, wie Kephaline oder Lecithine, Stärken, modifizierte Stärken oder Kohlenwasserstoffe, beispielsweise Vaseline oder Paraffine, dienen. Darüber hinaus können die Formulierungen Bestandteile enthalten, die selbst pharmazeutisch aktiv sind, wie beispielsweise die genannten Aldehyde und die genannten α-Hydroxycarbonsäuren oder Mittel, die für ihre Herstellung notwendig ist, z.B. die genannten Tenside.

Im Vordergrund steht hier die topische Applikation der erfindungsgemäßen Mittel. Die erfindungsgemäßen Komplexe lassen sich in Pudern, Salben, Cremes oder Gelen für die Wundversorgung im human- oder veterinärmedizinischen Bereich oder für die Versorgung bakterieller Hautinfekte, beispielsweise der Akne vulgaris, formulieren. Weiterhin sind die erfindungsgemäßen Komplexe für Formulierungen als Zahnwässer, Zahnpasten oder Zahnpulver geeignet. Weitere topische Formulierungen sind antibakterielle Lippenstifte, Ohrentropfen und Vaginalsuppositorien. Auch lassen sich die erfindungsgemäßen Komplexe in Lösungen für die Spülung von Körperhöhlen zur Behandlung von Fisteln formulieren. Bei Verwendung löslicher Komplexe können diese zusammen mit Klebemitteln für Pflaster verfilmt werden. Die so hergestellten Pflaster weisen einen desinfizierenden Depoteffekt auf. Aufgrund der Kombination von desinfizierender und desodorierender Wirkung der erfindungsgemäßen Polymerkomplexe eignen sich diese auch für die Formulierung als Mittel zur Körperpflege. Da die erfindungsgemäßen Komplexe als Feststoffe vorliegen, ist ihre Formulierung für medizinische Anwendungen unproblematisch und kann auf übliche Weise erfolgen. Hinsichtlich der Formulierungsbestandteile bestehen im Prinzip keine Einschränkungen mit Ausnahme der Verträglichkeit mit den erfindungsgemäßen Komplexen.

In der Kosmetik ist die Formulierung der erfindungsgemäßen Komplexe als Creme, Salbe oder Gel, beispielsweise in der Haarkosmetik für die Haarfärbung oder Haarbleichung, und für die Depilation möglich. Auch der Einsatz in Shampoos, z.B. zur Schuppenbehandlung, in Seifen oder Pudersprays ist denkbar.

Eine weitere Anwendungsform der erfindungsgemäßen Komplexe besteht in der Entkeimung von Flüssigkeiten. So eignen sich beispielsweise die wasserunlöslichen Ausführungsformen der erfindungsgemäßen Komplexe als Filtereinsätze für die Entkeimung von Trinkwasser oder Getränken. Ein Einsatz in Filteranlagen für Schwimmbäder ist ebenfalls denkbar.

Eine dritte Anwendungsform der erfindungsgemäßen Komplexe ist die bakterizide Ausrüstung von Gegenständen. Da durch geeignete Wahl der Comonomere Polymerkomplexe mit maßgeschneiderten Löslichkeitseigenschaften zugänglich sind, können die erfindungsgemäßen Polymerkomplexe in die unterschiedlichsten Materialien, beispielsweise in Beschichtungsmassen, Tränklösungen oder Klebstoffdispersionen eingearbeitet werden. Es ist somit möglich, sowohl glatte Oberflächen als auch Gewebe, Vliesstoffe oder Wirrfaservliese oder aber auch verpreßte Zellstoffmaterialien mit einer bakteriziden Ausrüstung zu versehen. Beispiele hierfür sind die Auskleidung von Flüssigkeitstanks, z.B. in Trinkwassersystemen oder Fäkalientanks in Chemikalientoiletten. Ebenfalls können Schwämme, Handtücher, Kleidungsstücke, Bettwäsche, Gardinen und Vorhänge mit den erfindungsgemäßen Komplexen bakterizid ausgerüstet werden. Als Anwendung im medizinischen Bereich ist die Ausrüstung von OP-Handschuhen, Gesichtsmasken, Tamponaden, Pads oder Tupfern mit den erfindungsgemäßen Komplexen denkbar. Eine Ausrüstung von Einlagen, insbesondere im Bereich der adulten Inkontinenz oder für die Monatshygiene, Windeln, Sportschuhen, Badematten, Kopf- oder Nackenstützen ist möglich.

Die erfindungsgemäßen Komplexe eignen sich darüber hinaus zum Einsatz in Luftfiltern für Klimaanlagen. Kombinationen der erfindungsgemäßen Komplexe mit Adsorbenzien und Trockenmitteln können in der Haustierhygiene, beispielsweise als Streugut oder Auslagematerial für Käfige, Anwendung finden.

Die erfindungsgemäßen Komplexe lassen sich auch als Bestandteile in Reinigungsmitteln und/oder Desinfektionsmitteln verwenden. Beispiele hierfür sind einerseits Haushaltsreiniger oder Desinfektionsmittel, insbesondere für Arztpraxen und für den Krankenhausbereich, andererseits aber auch Reinigungsmassen oder Tabletten für die Gebißreinigung oder die Reinigung von Zahnspangen oder Lösungen zur Desinfektion von Kontaktlinsen.

Eine technische Anwendung der erfindungsgemäßen Komplexe ist ihre Wirkung als Radikalinitiatoren beispielsweise für die Polymerisation olefinisch ungesättigter Polymere.

Die folgenden Beispiele sollen die Erfindung erläutern, ohne sie jedoch einzuschränken.

### Beispiele

### Herstellung der Polymer-Wasserstoffperoxid-Komplexe (Beispiele 1 bis 5)

### Verwendete Komponenten

- Wasserstoffperoxid: 50 %ige wäßrige Lösung, stabilisiert mit 0.082 Gew.-% kolloidalem Silber; Handelsprodukt der Fa. Hungerbach GmbH, Mörsdorf.
- Polyvinylpyrrolidon K30: K-Wert 30 nach H. Fikentscher, Cellulose-Chemie 13 (1932) 18, 71.; Handelsprodukt der BASF.
- Polyvinylpyrrolidon, vernetzt: Crospovidone®, Handelsprodukt der BASF AG.
- Polyvinylcaprolactam: K-Wert 30
- Copolymer aus Vinylimidazol/Vinylpyrrolidon (VI/VP = 9:1) hergestellt nach folgender Vorschrift: In einer Rührapparatur mit aufgesetztem Rückflußkühler wurde ein Gemisch aus 9 Teilen N-Vinylimidazol (VI), 1 Teil N-Vinylpyrrolidon (VP), 0,3 Teilen N,N'-Divinylimidazolidon, 100 Teilen Wasser und 0,1 Teilen Natronlauge (5%ig) vorgelegt und unter Zusatz von 0,1 Teilen eines vernetzten, wenig quellbaren Polymerisats auf Basis von VI und/oder VP im Stickstoffstrom auf 70°C erhitzt. Bei dieser Temperatur wurde 6 h polymerisiert. Das erhaltene Fällungspolymerisat wurde abgesaugt, mit Wasser gründlich gewaschen und bei 60°C getrocknet. Man erhielt ein weißes, körniges Produkt in einer Ausbeute von 96,5%.
- Copolymer aus Ethylacrylat/Methacrylsäure (EA/MAS: 1/1): Kollicoat MAE30 D der BASF AG.

### Analytik

Der Wasserstoffperoxidgehalt der erfindungsgemäßen Polymerkomplexe wurde durch Titration mit Kaliumpermanganat bestimmt. Die Bestimmung des Metallgehalts der erfindungsgemäßen Polymerkomplexe erfolgte durch Atomabsorptionsspektrometrie. Der Wassergehalt der Polymerkomplexe wurde durch Karl-Fischer Titration bestimmt.

### Homogene Polymer-Wassertstoffperoxid-Silber-Komplexe (Beispiele 1-3)

### Beispiel 1 (Herstellung durch Sprühtrocknung)

In einem Trockenturm (D 900 mm; H 1400 mm) wurde mit Hilfe einer Zweistoffdüse bei einem Druck von 1,5 bar eine Lösung aus
- 500 g: Polyvinylpyrrolidon K30
- 153 g: Wasserstoffperoxid (50 %-ige wäßrige Lösung stabilisiert mit 0.126 g kollidalem Silber) und
- 1347 g: Wasser
versprüht. Die Trocknung erfolgte mittels Stickstoff bei 1 bar und einer Turmeingangstemperatur von 160°C sowie einer Turmausgangstemperatur von 70°C. Das entstandene Pulver wurde über einen Zyklon vom Gasstrom abgetrennt. Das erhaltene Pulver wies einen Peroxidgehalt von 12.9 Gew.-%, einen Silbergehalt von 0.019 Gew.-% und einen Wassergehalt von 3 Gew.-% auf.

### Beispiel 2 (Herstellung durch Sprühtrocknung)

150 g Polyvinylpyrrolidon und 5.6 g Silbernitrat wurden in 500 ml Ethanol gelöst und 60 min zum Rückfluß erhitzt. Anschließend gab man 60 g einer 25 %igen, wäßrigen Wasserstoffperoxid-Lösung zu. Die erhaltene Lösung wurde wie in Bsp. 1 sprühgetrocknet. Das erhalten Pulver wies einen Peroxidgehalt von 8 Gew.-%, einen Silbergehalt von 1,9 Gew.-% und einen Lösemittelgehalt von 1 Gew.-% auf.

### Beispiel 3 (Herstellung durch Wirbelbettrocknung)

Die Herstellung durch Wirbelbettrocknung erfolgt in einem Granulierzylinder, der nach unten durch einen Lochboden mit Siebauflage (Maschenweite 10-500 µm) und nach oben durch 4 Filtersäcke, welche alle 15 sec durch Druckluf freigeblasen werden, abgeschlossen ist. 28 cm oberhalb des Siebbodens befindet sich eine dem Siebboden entgegengerichtete Zweistoffdüse. Die Dosierung der Wasserstoffperoxid-Silber-Lösung erfolgt mittels einer Schlauchpumpe und Zugabegeschwindigkeiten von 2,5 bis 100 g/min/1000 g Polymer. Die Einsatzmenge an Polymer beträgt 100 bis 4000 g. Der Gasdurchsatz wird durch eine Abluftklappe reguliert und beträgt 120 m³/h bis 150 m³/h. Als Pozessgas wird Stickstoff verwendet. Die Zulufttemperatur liegt im Bereich von 25 bis 80°C, die Ablufttemperatur im Bereich von 25 bis 70°C.

250 g Polyvinylcaprolactam wurden bei einem Gasstrom von 120 m³/h im Wirbelbett vorgelegt und bei 50°C mit 153 g einer 50 Gew.-%igen wäßrigen Wasserstoffperoxid-Lösung, die 0,126 g kolloidales Silber enthielt, besprüht (10 g/min). Anschließend wurde im Gasstrom bei 50°C (150 m³/h; 20 min) getrocknet. Der Peroxidgehalt des erhaltenen Pulvers betrug 23 Gew.-%, der Silbergehalt 0,035 Gew.-% und der Wassergehalt 1 Gew.-%.

### Schalenförmige Polymer-Wasserstoffperoxid-Silber-Komplexe (Beispiele 4 bis 7)

### Beispiel 4 (VI/VP-Wasserstoffperoxid-Silber-Komplex)

200 g unlösliches VI/VP-Polymerisat wurden in einem Wirbelschichtgranulator nach Bsp. 3 vorgelegt und bei 60°C in 4 Intervallen mit jeweils 25 ml und dann in 3 Intervallen mit jeweils 50 ml 20 %igem Wasserstoffperoxid (jew. 20 ml/min) besprüht. Zwischen den Sprühintervallen wurde jeweils 5 min. im Gasstrom getrocknet. Auf die gleiche Weise wurde auf den erhaltenen Polymer-Wasserstoffperoxidkomplex 250 ml einer wäßrigen Silberkolloidsuspension mit einem Silbergehalt von 0.16 Gew.% gesprüht. Der erhaltene Komplex wies einen Peroxidgehalt von 16,8 Gew.-% einen Silbergehalt von 0.196 Gew.-% und einen Wassergehalt von 3,7 Gew.-% auf.

### Beispiel 5 (Polymer-Wasserstoffperoxid-Silber-Komplex mit Filmbildner) 100 g vernetztes Polyvinylpyrrolidon wurden zunächst wie in Beispiel 4 mit 200 ml einer 15 %-igen Wasserstoffperoxidlösung besprüht. Die erhaltenen Polymer-Wasserstoffperoxid-Komplexe besprühte man in 4 Portionen mit einer Lösung aus 15 g Kollicoat MAE 30 D, 2g Triethylcitrat und 0,1 g kolloidalem Silber in 100 ml Wasser und trocknete wie in Beispiel 3. Der erhaltene Komplex wies einen Peroxidgehalt von 18 Gew.-%, einen Silbergehalt von 0,06 Gew.-% und einen Wassergehalt von 2 Gew.-% auf. Silber und Wasserstoffperoxid wurden bei einem pH-Wert von 5,5 freigesetzt.

### Beispiel 6

100 g vernetztes Polyvinylpyrrolidon wurden zunächst wie in Beispiel 4 mit 200 ml einer 15 %-igen Wasserstoffperoxidlösung besprüht. Die erhaltenen Polymer-Wasserstoffperoxid-Komplexe besprühte man in 4 Portionen mit einer Lösung aus 15 g Kollicoat MAE 30 D und 2g Triethylcitrat. Anschließend wurde mit einer Lösung aus 0,1 g kolloidalem Silber in 100 ml Wasser besprüht und wie in Beispiel 3 getrocknet.

### Beispiel 7

Es wurde wie in Beispiel 6 verfahren, jedoch wurde anstelle des Silberkolloids eine 0,1 Gew.-%ige Lösung von Silbernitrat verwendet. Der Silbergehalt im erhaltenen Komplex betrug 0,04 Gew.-%.

### Formulierungden der erfindungsgemäßen Polymerkomplexe (Beispiele 8 bis 12)

verwendete Komponenten
- Polyacrylsäure: Carbopol® C981, der Fa. BF Goodrich Chemical
- Ethylenoxid/Propylenoxid-Blockcopolymer (EO/PO 70/30) Mₙ 9840 bis 146000; Lutrol® F 127 der BASF.
- Polyethylenglykol: Mₙ 400; Lutrol® E 400 der BASF.
- Polyethylenglykol: Mₙ 4000; Lutrol® E 4000 der BASF.
- Silkonöl: Dichte ρ(25°C) 0,95 g/cm³, Viskosität (25°C) 2,5 mm²/s, Dow Corning Fluid 344 (cyclisches Tetra(dimethyl)siloxan der Dow-Corning.
- Distärkeposphat auf Basis von Maisstärke: Mais PO4 100 K (P-Gehalt: < 0,1 Gew.-% in Trockenmasse); Fa. Hauser KG

### Beispiel 8 (Formulierung als Zahncreme)

10 g des nach Beispiel 1 hergestellten Komplexes wurden in 78 g Wasser gelöst und in einem Vakuumhomogenisator mit 2 g Carbopol® und 10 g 1,2-Propylenglykol zu einem luftblasenfreien Gel verarbeitet.

### Beispiel 9 (Gel zur Hautdesinfektion)

10 g Komplex aus Beispiel 1, 75 g Wasser, 5 g 1,2-Propylenglykol und 20 g Lutrol® F127 wurden unterhalb 10°C in der für Beispiel 6 beschriebenen Weise zu einem Gel verarbeitet.

### Beispiel 10 (Formulierung als Salbe)

20 g Komplex aus Beispiel 1 wurden in einer Mischung aus 50 g Lutrol® E 400 und 5 g Wasser gelöst und auf 55-60°C erwärmt. Anschließend arbeitete man bei dieser Temperatur unter Rühren 25 g Lutrol® E 4000 ein und ließ unter Rühren abkühlen.

### Beispiel 11 (Formulierung als Konzentrat für Mundspülungen)

25 g Komplex aus Beispiel 1 wurden in einer Mischung aus 1 g 1,2-Propylenglykol, 9 g Ethanol und 65 g Wasser gelöst.

### Beispiel 12 (Formulierung als Puderspray)

2,5 g Komplex aus Beispiel 1 wurden in einem Mikronisator mikronisiert und zusammen mit 1 g Silikonöl (s.o.) und 2,5 g Maisstärke-Diphosphat in ein Druckgefäß gegeben. Anschließend befüllte man mit 5 g Pentan und 2,2 g Propan/Butan.

## Patentansprüche

1. Polymerkomplexe, enthaltend als wesentliche Bestandteile
a) Wasserstoffperoxid,
b) ein zur Komplexbildung mit Wasserstoffperoxid geeignetes Polymer und
c) wenigstens ein Metallkolloid oder Metallsalz.

2. Polymerkomplexe nach Anspruch 1, in denen der Bestandteil b) ein Polymer auf Basis von N-Vinyllactamen ist.

3. Polymerkomplexe nach Anspruch 2, in denen der Bestandteil b) aufgebaut ist aus
20 bis 100 Gew.-% wenigstens eines N-Vinyllactams,
0 bis 80 Gew.-% wenigstens eines copolymerisierbaren, monoethylenisch ungesättigten Monomeren und
0 bis 20 Gew.-% wenigstens eines vernetzend wirkenden Monomeren.

4. Polymerkomplexe nach Anspruch 2 oder 3, in denen das N-Vinyllactam ausgewählt ist unter N-Vinylpyrrolidon, N-Vinylpiperidon, N-Vinylcaprolactam, N-Vinylmorpholin-3-on, N-Vinyloxazolidin-4-on.

5. Polymerkomplexe nach einem der vorhergehenden Ansprüche, in denen der polymere Bestandteil b) ein Homo- oder Copolymer des N-Vinylpyrrolidons oder des N-Vinylcaprolactams mit einem K-Wert im Bereich von 10 bis 110 ist.

6. Polymerkomplexe nach einem der vorhergehenden Ansprüche, in denen das Metall ausgewählt ist unter Cu, Ag, Au, Rh, Ir, Pd, Pt.

7. Polymerkomplexe nach einem der vorhergehenden Ansprüche, in denen der Bestandteil c) ein Silberkolloid oder Silbersalz ist.

8. Polymerkomplexe nach einem der vorhergehenden Ansprüche mit schalenförmigem Aufbau, wobei die Komplexe auf einem Kern angeordnet sind.

9. Polymerkomplexe nach Anspruch 8, wobei der Kern ein vernetztes, wasserunlösliches, zur Komplexbildung mit Wasserstoffperoxid geeignetes Polymer ist.

10. Polymerkomplexe nach Anspruch 8 oder 9 mit mehrschaligem Aufbau, wobei der Kern aus der Polymer b) mit den Komponenten a) und/oder c) und die Schalen aus der Polymer b) mit den Komponenten a) und/oder c) oder aus einem polymeren Filmbildner bestehen.

11. Polymerkomplexe nach einem der vorhergehenden Ansprüche, enthaltend
Bestandteil a) in einer Menge von 0,5 bis 40 Gew.-%,
Bestandteil b) in einer Menge von 55 bis 99,5 Gew.-%,
Bestandteil c) in einer Menge von 0,001 bis 5 Gew.-%,
jeweils bezogen auf das Gesamtgewicht der Polymerkomplexe.

12. Verfahren zur Herstellung der Polymerkomplexe der Ansprüche 1 bis 11, dadurch gekennzeichnet, daß man die Bestandteile a), b) und c) miteinander in Kontakt bringt oder gegebenenfalls auf einen Kern aufbringt.

13. Verwendung der Polymerkomplexe aus einem der Ansprüche 1 bis 11 zur Herstellung von Arzneimitteln mit bakterizider Wirkung, zur bakteriziden Ausrüstung von Gegenständen und Zubereitungen, zur Entkeimung wäßriger Flüssigkeiten, in haarkosmetischen Mitteln oder als radikalischer Initiator für chemische Reaktionen.

14. Mittel, enthaltend wenigstens einen der Polymerkomplexe aus einem der Ansprüche 1 bis 11.

15. Mittel nach Anspruch 14, das in Form eines kosmetischen oder pharamzeutischen Mittels oder in Form eines Mittels zur Desinfektion von wäßrigen Flüssigkeiten und Gegenständen vorliegt.
